# EUROPEAN PATENT SPECIFICATION

(11) **EP 4 639 158 B1**
(45) Date of publication and mention of the grant of the patent: **18.11.2026**
(21) Application number: 23833466.8
(22) Date of filing: 18.12.2023
(51) Int. Cl.: G01N 25/18, G01N 33/24

(54) **METHOD AND SYSTEM FOR DETERMINING THERMAL PROPERTIES OF SOIL FIELD**
VERFAHREN UND SYSTEM ZUR BESTIMMUNG DER THERMISCHEN EIGENSCHAFTEN EINES BODENFELDS
PROCÉDÉ ET SYSTÈME DE DÉTERMINATION DE PROPRIÉTÉS THERMIQUES DE CHAMP DE TERRAIN

(30) Priority: 22.12.2022 NL 2033804
(43) Date of publication of application: 29.10.2025
(73) Proprietor: FNV IP B.V., 2264 SG Leidschendam (NL)
(72) Inventor: VARDON, Phil, 2264 SG Leidschendam (NL); DANIILIDIS, Alexandros, 2264 SG Leidschendam (NL); KOVALENKO, Vsevolod, 2264 SG Leidschendam (NL); VRIELINK, Leon, 2264 SG Leidschendam (NL); PARASIE, Nico, 2264 SG Leidschendam (NL); LOOIJEN, Peter, 2264 SG Leidschendam (NL); KROHOF, Stan, 2264 SG Leidschendam (NL); VAN ZANTEN, Sophie, 2264 SG Leidschendam (NL)
(74) Representative: FNV Intellectual Property
(86) International application number: PCT/EP2023/086444
(87) International publication number: WO 2024/133133

(56) References cited:
- CN-A- 112 485 291
- DE-A1- 102011 001 153
- US-A1- 2006 191 683
- MO PIN-QIANG ET AL: "Interpretation of heating and cooling data from thermal cone penetration test using a 1D numerical model and a PSO algorithm", COMPUTERS AND GEOTECHNICS, ELSEVIER, AMSTERDAM, NL, vol. 130, 14 November 2020 (2020-11-14), XP086423683, ISSN: 0266-352X, [retrieved on 20201114], DOI: 10.1016/J.COMPGEO.2020.103908

## Description

The present disclosure relates generally to a method and system for determining the thermal properties of soil. More particularly, the present disclosure relates to a method and system for determining subsurface thermal conductivity and volumetric heat capacity using a heat flow penetrometer. Unlocking insights from Geo-Data, the present invention further relates to improvements in sustainability and environmental developments: together we create a safe and liveable world.

### BACKGROUND

There is a general and ongoing need to improve data acquisition of subsurface surveying. The determination of subsurface characteristics is used to identify objects below the surface of the ground, as well as determining the soil characteristics, such as soil type, density, moisture content, shear modulus, and the like, which may be used in foundation planning and/or management. Subsurface information may be used for e.g., site characterisation for infrastructure projects, foundation calculations, and the like. For such applications, it is important to generate a comprehensive understanding of the subsurface with a high degree of accuracy in an efficient manner.

One of the methods of performing such tests is generally known as a cone penetration test (CPT). The cone penetration is a geotechnical investigation method for determining, e.g., soil and groundwater characteristics, where a cone penetrometer is pushed into the soil to perform a measurement or measurements. Typical parameters measured by such a probe are cone tip resistance, sleeve friction and pore-water pressure. Usually, the test method comprises pushing an instrumented cone penetrometer probe, with the tip facing down, into the ground at a controlled rate.

There is a need to determine thermal characteristics of the soil. One such characteristic is thermal conductivity of the soil, which provides a measure of energy dissipation through soil. The thermal conductivity of a material is a measure of its ability to conduct heat and is defined as the transport of energy due to random molecular motion across a temperature gradient. This is an important characteristic to determine since it influences fundamental aspects of infrastructure construction and other subsurface activities, such as foundation design, cable routing and cable design, insulation properties of subsurface structures, and the like. The measure of heat conduction also influences, for example, how fast heat produced in power and/or data cables can dissipate so as to stop such cables from overheating. Optimal cable routing may also be influenced by a measure of heat dissipation such that a route is chosen in which the thermal conductivity of the soil does not negatively influence the operating characteristics of the cable. Thermal characteristics of the soil may be similarly relevant for other applications.

Known methods of performing such a CPT utilise a cone penetrometer assembly built from a plurality of rod segments, forming a string of rods with a cone penetrometer positioned at the tip which is able to measure at the required depth. Other known methods utilize coiled CPT systems in which an elongated pushrod is used, in which the pushrod bent into a coiled shape such that the pushrod may be straightened prior to being advanced into the soil. Conventional CPT systems do not provide the possibility of measuring thermal conductivity. Cone penetrometers and their uses are discussed in "Interpretation of heating and cooling data from thermal cone penetration test using a 1D numerical model and a PSO algorithm", MO PIN-QIANG ET AL, COMPUTERS AND GEOTECHNICS, ELSEVIER, AMSTERDAM, NL, vol. 130, 14 November 2020 (2020-11-14),ISSN: 0266-352X, DOI: 10.1016/J.COMPGEO.2020.103908 and CN 112 486 291.

Attempts have been made to produce CPT systems that allow for the determination of thermal conductivity. A known CPT system comprises a cone penetrometer having a friction sleeve, positioned between a cone and a pushrod, which is arranged to be heated through friction heating as the CPT system is advanced into the soil. As the CPT system is advanced into the soil, the cone penetrometer heats up storing an amount of energy. Once the cone penetrometer has achieved a certain temperature, the propagation of the CPT system is halted, so that no further friction-induced energy is provided to the system. At this point, the cone penetrometer gives off heat to the surrounding soil as the temperature of the cone penetrometer is higher than the temperature of the surrounding soil. The speed with which the temperature of the cone penetrometer reduces is indicative of the thermal conductivity of the soil surrounding the CPT system. If the soil has a higher thermal conductivity, the heat dissipates at a higher rate, leading to a higher average temperature differential and as a result a higher reduction of temperature of the cone penetrometer. A thermometer is generally positioned in the cone of the cone penetrometer, which measures the decrease of the temperature after the cone and friction sleeve have heated through friction with the soil.

In particular, the dissipation of thermal energy through the soil, i.e., thermal conductivity, determines the speed with which the temperature gradient between the cone penetrometer and the soil is reduced. If the soil heats up but cannot dissipate the energy quickly, the temperature of the soil directly surrounding the cone penetrometer will rise, such that the temperature differential between the cone penetrometer and the soil is reduced and the rate of the temperature reduction of the cone penetrometer flattens. As a result, the temperature decline of the cone penetrometer is less steep, providing information on a low thermal conductivity of the soil. Similarly, if the rate at which the energy dissipates is very high, i.e., the rate of temperature reduction of the cone penetrometer is high, the thermal conductivity of the surrounding soil is high.

A problem associated with the known cone penetrometers is that soil-friction-induced heating generally requires a temperature change of at least 3°C, which corresponds approximately to at least 3 MPa cone resistance as being advanced at least about 1 m through the soil. If a lower cone resistance is provided by the soil, the required temperature change to perform an accurate measurement is not achieved. As a result, such passive CPT systems cannot be used in softer soils as these do not provide sufficient cone resistance and thus insufficient heat generation. Further, even if sufficient heat is generated over a longer depth, this reduces the measurement resolution of the CPT system, as it requires a longer travel path into the soil to generate sufficient heat to perform the test, meaning the thermal properties soil cannot be determined at shallower depths. In addition, as the CPT system only comprises a single thermometer in the cone of the CPT system, the time needed to perform testing over a relevant section of soil is long.

To mitigate the problem of measuring thermal conductivity in softer soils, a known approach is to utilize a heat flow needle. This is a thin, elongated needle provided at the tip of an in-situ probe. The heat flow needle is actively heated and is thus not dependent on friction-induced heating. A needle is used since it defines an aspect ratio of length (L) with respect to its diameter (D) which allows the thermal conductivity to be modelled under the assumption that the needle is defined as an infinite line heat source model, with accuracy sufficient for determination of the soil parameters. In other words, the needle is considered to act as a source of heat that is infinitely long and has a negligible cross section, known as infinite line heat source. The heat transfer equation for such an infinite line source in homogeneous soil allows for an analytical solution as the direction of heat dissipation is a one-dimensional problem. In other words, the heat dissipation is homogeneous in all directions, both radial and axial. The analytical form of this solution allows for use of its inversion to obtain the thermal parameters of the soil using a relatively short measurement time.

The analytical solution to energy dissipation can be inverted such that a value for thermal conductivity of the soil can be obtained from the temperature measurements of the heat flow needle probe. The analytical solution assumes that there is an infinite line heating element with a constant heat input at r = 0. However, the needle must have a sufficiently small diameter-length ratio (D/L-ratio) since it otherwise does not adhere to an infinite line heat source model. If the D/L-ratio is large, it will no longer be possible to use an infinite line heat source model as this will lead to errors in the calculated thermal conductivity. In particular, a shorter D/L ratio means that isotherms (lines with a constant temperature), which are curved at the ends of the needle, have a greater influence on the overall heat dissipation. As a result of the influence of the curved isotherms on the overall heat dissipation, the directional heat dissipation can no longer be assumed homogeneous along the needle. Further, the minimum amount of time needed to measure with the heat flow needle probe method (taking the log-linear gradient of the infinite line heat source model) directly relates to the diameter of the heat flow needle used. The larger the diameter of the heat flow needle, the more time is needed to get an accurate measurement. Time scales of measurement may be roughly correlated with radius of the heat flow needle, when considered for the infinite line heat source model, according to the following condition: $t > \frac{\left(5∗C∗{r}^{2}\right)}{k}$ (1) where
t is time
r is radius of the heat flow needle (diameter/2)
C is volumetric heat capacity
k is thermal conductivity

As an example, for a heat flow needle with a radius of around 28 mm, a measurement could take over an hour, assuming a volumetric heat capacity of 2.2 MJ/m³K and a thermal conductivity of 2.5 W/mK. However, a second problem is that the diameter-length ratio must be sufficiently small to adhere to an infinite line heat source model. A deviation from the infinite line source model even further increases the required measurement time to attain any useable measurement results.

Due to its small diameter, necessary for modelling the needle as an infinite line, the heat flow needle is fragile and cannot be used in harder soils. Also, as with the friction-induced thermal CPT system, the single heat flow needle takes a long time to measure soil characteristics over a depth range. Since the needle can only be positioned at the tip of the tool, it must be moved periodically to different depths in order to obtain measurements over a depth range.

Further, if a larger diameter of measurement probe than a heat flow needle probe were to be used to measure temperature, a much longer the measurement time would be required in order for the one-dimension analytical solution to provide an accurate result for thermal conductivity for the reasons outlined above. Such measurement times are often infeasible in practical applications of soil characteristic determination of the type mentioned above. Further, if the infinite line heat source model interpretation approach would be used on data obtained from a larger diameter-length ratio measurement probe, this would result in significant errors in thermal conductivity predictions.

Since the heat flow needle probe cannot be utilized in harder soils, a hard layer overlaying a soft layer cannot be measured. That is, the soft layer underneath may be reached by the more robust friction sleeve probe, but a system using a friction sleeve probe cannot take measurements in the soft soil because the friction-induced heat is too low. At the same time, the heat flow needle probe cannot penetrate the hard soil to begin with and can thus not reach the soft soil underneath. An option is to create a borehole to advance the needle into the soft soil through the drilled hole. However, this is very expensive and inefficient. A better solution is desirable.

As a result of the above, there is a need for improved methods and systems for determining thermal properties of soil in which a more robust measurement probe can be used at the same time as providing a determination of thermal properties in a faster timeframe. It would be advantageous to provide systems and methods which address one or more of the above problems, in isolation or in combination.

### OVERVIEW

According to a first aspect of the present disclosure, there is provided computer-implemented method of determining subsurface thermal conductivity of target soil. The method comprises receiving, from a heat flow penetrometer that is surrounded by target soil, temperature data comprising a temperature at each time point of n time points, determining, for each time point a measured temperature difference, and, based on estimated values of thermal conductivity and volumetric heat capacity of the target soil, using a solution space to generate a calculated temperature difference for the estimated values of thermal conductivity and volumetric heat capacity for each of a predetermined series of time points of the solution space. The solution space comprises a plurality of values of thermal conductivity, a plurality of values of volumetric heat capacity, and pre-computed values of temperature difference for each combination of values of thermal conductivity and volumetric heat capacity and for each of the predetermined series of time points of the solution space. The method further comprises comparing the measured temperature difference and the calculated temperature difference for each time point of the solution space to determine an error value for the estimated values of thermal conductivity and volumetric heat capacity, adjusting the estimated values of thermal conductivity and volumetric heat capacity of the target soil based on the error value, and outputting the adjusted estimated values of thermal conductivity and volumetric heat capacity.

The method makes enables the accurate estimation of values of thermal conductivity and volumetric heat capacity for target soil, where an analytical solution is not feasible, to be carried out in a computationally efficient manner. Instead of directly running a numeric forward model to estimate thermal conductivity and volumetric heat capacity each time a measurement of temperature is made using a heat flow penetrometer, the precomputed values of temperature difference stored in the solution space are used. This is more computationally efficient than directly running a numeric forward model each time a measurement of temperature is made using a heat flow penetrometer.

The aforementioned need for long measurement times associated with the use of larger diameter measurement probes when using an analytical solution are also addressed. The method can be used for larger diameter measurement probes without the measurement times associated with using an analytical solution.

Further, the aforementioned issue for larger D/L-ratio measurement probes, where it is no longer be possible to use an infinite line heat source model as this will lead to errors in the calculated thermal conductivity, is addressed. The method can be used for larger D/L-ratio measurement probes without such issues arising.

In some implementations, the additional step of normalizing each measured temperature difference, Δ*T,* based on a reference power level, *Q_{ref},* and a power level at which the temperature data is generated, *Q_{data}* is provided.

This step means that the power level at which the temperature data received from the heat flow penetrometer is generated is normalised to that of the solution space. Accordingly, it enables the power level at which the temperature data from the heat flow penetrometer is generated to be different to that at which the pre-computed values of temperature difference of solution space were generated.

In some implementations, the estimated values of thermal conductivity, *k,* and volumetric heat capacity, *C,* are adjusted using a minimization solver to minimize the error value.

In this manner, the estimated values of thermal conductivity, *k,* and volumetric heat capacity, *C,* are adjusted find a better match with the temperature data received from the heat flow penetrometer.

In some implementations, a regularization function is added to the minimization solver which provides a penalty when an adjusted estimated volumetric heat capacity, *C,* is generated that differs from a predetermined volumetric heat capacity, *C.*

In this manner, the sensitivity of the method to the determination of volumetric heat capacity is reduced by using a regularization function, improving accuracy of the minimization solver and, ultimately the matching of the estimated values of thermal conductivity, *k,* and volumetric heat capacity, *C,* with the temperature data received from the heat flow penetrometer.

In some implementations, the regularization function is a quadratic loss function.

In some implementations, the pre-computed values of temperature difference, Δ*T,* are pre-computed using a numerical forward solver.

In some implementations, the pre-computed values of temperature difference, Δ*T,* are pre-computed based on at least one geometrical characteristic of the heat flow penetrometer.

In some implementations, the at least one geometrical characteristic of the heat flow penetrometer comprises a simplified geometry of the heat flow penetrometer.

In this manner, the computational overhead required to generate the pre-computed values is reduced.

In some implementations, the simplified geometry is that of an axisymmetric solid probe.

In some implementations, the pre-computed values of temperature difference, Δ*T,* are pre-computed based on a thermal characteristic of the heat flow penetrometer. Example thermal characteristics include one or more of: shape/geometry, weight, density, thermal conductivity, volumetric heat capacity of one or more of the constituent materials/elements of the heat flow penetrometer.

In some implementations, the measured temperature difference, Δ*T,* for each time point, *tⱼ,* is determined by subtracting an initial temperature, *T*₀ of the heat flow penetrometer from the temperature, *T*, at each time point, *tⱼ*.

In some implementations, the measured temperature difference, Δ*T,* for each time point, *tⱼ,* is determined with respect to the temperature of a previous time point, *t*_{*j*-1}, for example, by subtracting the previous temperature, *T*_{*tj-*1}*,* from the temperature, *Tₜⱼ,* for each time point *tⱼ.*

In some implementations, the additional step of matching the measured temperature difference, Δ*T,* to the solution space based on the predetermined series of time points, *tᵢ* of the solution space and using an interpolation, such as a, linear interpolation is provided.

This normalises the time period used for measurement of the temperature data by the heat flow penetrometer to the time period of the solution space. This means that the time period used for measurement of the temperature data by the heat flow penetrometer does not need to match that of the solution space.

In some implementations, the error value is determined by calculating the root mean square error of the measured temperature difference, Δ*T ,* and the calculated temperature difference, Δ*T̂,* for each time point, *tᵢ*, of the solution space.

In some implementations, the solution space comprises a three-dimensional array with the plurality of values of thermal conductivity, *k,* on the x-axis, the plurality of values of volumetric heat capacity, *C,* on the y-axis, the predetermined series of time points, *tᵢ*, on the z-axis, and the pre-computed values of temperature difference, Δ*T,* stored as data points in the three-dimensional array.

In some implementations, the step of using a solution space to generate a calculated temperature difference, Δ*T̂,* for the estimated values of thermal conductivity, *k,* and volumetric heat capacity, *C,* for each of a predetermined series of time points, *tᵢ*, of the solution space comprises using a two-dimensional interpolator to interpolate the pre-computed values of temperature difference, Δ*T,* stored as data points to find the calculated temperature difference, Δ*T̂,* at each of a predetermined series of time points, *tᵢ*.

In this manner, the calculated temperature differences, Δ*T̂,* can be obtained in a computationally efficient manner using a two-dimensional interpolator.

According to another aspect of the present disclosure, there is provided a computer apparatus configured to perform any of the methods disclosed herein.

According to another aspect of the present disclosure, there is provided a system comprising one or more processors and one or more memories having stored thereon computer-readable instructions configured to cause the one or more processors to perform any of the methods disclosed herein.

According to another aspect of the present disclosure, there is provided the computer apparatus or system above and a heat flow penetrometer.

According to another aspect of the present disclosure, there is provided a computer-readable medium comprising instructions, that, when executed by one or more data processing apparatus, cause the one or more data processing apparatus to perform any of the methods disclosed herein.

According to another aspect of the present disclosure, there is provided a computer program comprising instructions which, when the program is executed by a computer, cause the computer to perform any of the methods disclosed herein.

### BRIEF DESCRIPTION OF DRAWINGS

The exemplary embodiments will hereinafter be described in conjunction with the following drawing figures, wherein like numerals denote like elements, and wherein:
FIG. 1 is a side view of a heat flow cone penetrometer system;
FIG. 2 is a cut-out view of the heat flow cone penetrometer system;
FIG. 3 is a three-dimensional view of the inner sleeve of a heat flow module in the heat flow cone penetrometer system;
FIG. 4 is a three-dimensional cross-sectional view of a section of the heat flow cone penetrometer system showing the heat flow module;
FIG. 5 is an example heat flow needle probe 51;
FIG. 6 is a schematic overview of the process of constructing a solution space;
FIG. 7 is a schematic overview of some steps of the inversion method;
FIG. 8 is a schematic overview of some steps of the inversion method;
FIG. 9 is a flowchart of a method, implemented on a computer, of determining subsurface thermal conductivity of target soil; and
FIG. 10 is a block diagram of one implementation of a computing device.

### DETAILED DESCRIPTION

The following is a description of certain aspects of the disclosure, given by way of example only and with reference to the drawings.

Various aspects of the disclosure are discussed in detail below. While specific implementations are discussed, it should be understood that this is done for illustration purposes only. A person skilled in the relevant art will recognize that other components and configurations may be used without parting from the scope of the disclosure. Thus, the following description and drawings are illustrative and are not to be construed as limiting. Numerous specific details are described to provide a thorough understanding of the disclosure. However, in certain instances, well-known or conventional details are not described in order to avoid obscuring the description. A reference to an aspect in the present disclosure can be a reference to the same aspect or any other aspect. Such references thus relate to at least one of the aspects herein.

Reference to "one aspect" or "an aspect" means that a particular feature, structure, or characteristic described in connection with the aspect is included in at least one aspect of the disclosure. The appearances of the phrase "in one aspect" in various places in the specification are not necessarily all referring to the same aspect, nor are separate or alternative aspects mutually exclusive of other aspects. Moreover, various features are described which may be exhibited by some aspects and not by others.

The terms used in this specification generally have their ordinary meanings in the art, within the context of the disclosure, and in the specific context where each term is used. Alternative language and synonyms may be used for any one or more of the terms discussed herein, and no special significance should be placed upon whether or not a term is elaborated or discussed herein. In some cases, synonyms for certain terms are provided. A recital of one or more synonyms does not exclude the use of other synonyms. The use of examples anywhere in this specification including examples of any terms discussed herein is illustrative only and is not intended to further limit the scope and meaning of the disclosure or of any example term. Likewise, the disclosure is not limited to various aspects given in this specification.

Without intent to limit the scope of the disclosure, examples of instruments, apparatus, methods, and their related results according to the aspects of the present disclosure are given below. Note that titles or subtitles may be used in the examples for convenience of a reader, which in no way should limit the scope of the disclosure. Unless otherwise defined, technical and scientific terms used herein have the meaning as commonly understood by one of ordinary skill in the art to which this disclosure pertains. In the case of conflict, the present document, including definitions will control.

Additional features and advantages of the disclosure will be set forth in the description which follows, and in part will be obvious from the description, or can be learned by practice of the herein disclosed principles. The features and advantages of the disclosure can be realized and obtained by means of the instruments and combinations particularly pointed out in the appended claims. These and other features of the disclosure will become more fully apparent from the following description and appended claims or can be learned by the practice of the principles set forth herein.

### Description of heat flow cone penetrometer system

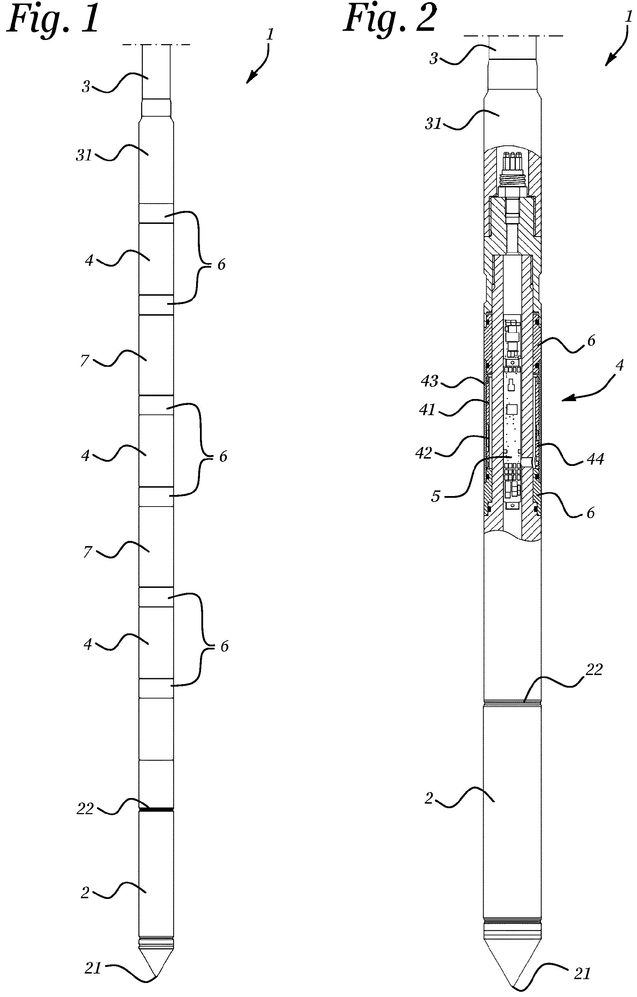

Referring to FIG. 1, a side view of the heat flow cone penetrometer system 1 according to an example is shown. The heat flow cone penetrometer system 1 shown in FIG. 1 is merely presented an example of a heat flow penetrometer. It will be understood that where ever reference is made to a heat flow cone penetrometer system 1, a more generally defined heat flow penetrometer could be used. Such a heat flow penetrometer does not necessarily have to be a heat flow cone penetrometer system 1. While it is useful to measure cone resistance, pore pressure etc., simultaneously, a heat flow penetrometer may also be a penetrometer with a dummy tip having no other measurement capabilities beyond measuring temperature of target soil. In its most fundamental form, heat flow penetrometer could be a penetrometer with a heating element and a temperature measuring element, such as a thermometer.

The heat flow cone penetrometer system 1, which is one example of a heat flow penetrometer 1, is arranged to measure subsurface thermal conductivity. The heat flow cone penetrometer system 1 comprises a cone penetrometer 2. The cone penetrometer may be a standard cone penetrometer commonly used in industry, or a penetrometer system having a dummy tip with no other measurement capabilities beyond temperature measurement. The cone penetrometer 2 extends between a tip 21 and a proximal end 22. The heat flow cone penetrometer system 1 further comprises a pushrod 3, which is arranged to translate a compressive force to the cone penetrometer 2 as it is being driven into a target soil. The pushrod 3 comprises a connection section 31. The connection section 31 of the pushrod 3 is arranged to couple pushrod 3 to components of the heat flow cone penetrometer system 1.

The heat flow cone penetrometer system 1 in the shown example comprises three heat flow modules 4, separated by intermediate sections 7. The heat flow modules 4 are provided between the proximal end 22 of the cone penetrometer 2 and the connection section 31 of the pushrod 3. Each of the heat flow modules 4 of the illustrated example comprises at least one heating element 41 and at least one temperature sensor 42 (both shown in FIG. 2).

Between the heat flow modules 4 in the shown example, a plurality of insulation rings 6 are provided. These insulation rings 6 are arranged to insulate the heat flow modules from one another, such that the heat produced by a first heat flow module 4 does not affect the temperature measurements of the temperature sensor 42 of a second heat flow module 4.

The heat flow modules 4 are axially stacked between the cone penetrometer 2 and the connection section 31 of the pushrod 3. The heat flow cone penetrometer system 1 may comprise more or fewer heat flow modules 4 than are depicted in FIG. 1, as may be appropriate to achieve sufficient resolution of thermal dissipation in the target soil, and/or to increase the total range of measurement depth in one measurement cycle.

Now referring to FIG. 2, a cut-out view of the heat flow cone penetrometer system 1 according to an example is shown. For illustrative purposes, FIG. 2 shows only a single heat flow module 4 between the connection section 31 of the pushrod 3 and the proximal end 22 of the cone penetrometer 2. The heat flow modules 4 depicted in FIG. 1 may have the same features as the heat flow module 4 depicted in FIG. 2 and described here.

The heat flow module 4 comprises a dissipation sleeve 43 and an inner sleeve 44. The dissipation sleeve 43 is provided around the inner sleeve 44. The inner sleeve 44 comprises the heating element 41. In this case, the heating element 41 is a heating wire, which is spooled around the inner sleeve 44 through grooves 47 provided on an outer surface of the inner sleeve 44.

The heat flow module 4 further comprises a temperature sensor 42, which is contained in a slot on the outer surface of the inner sleeve 44. The heat generated by the heating element 41 in the form of a heating wire 41 provided in the grooves 47 on the outer surface of the inner sleeve is distributed through the inner sleeve 44 and the dissipation sleeve 43, which is provided around the inner sleeve 44. Once the measurement is started, the heat production through the heating wire 41 is halted, after which the temperature sensor 42 measures the decline in temperature as heat dissipates from the inner sleeve into the soil. In an alternative example, the measurement is performed during the heating of the heating wire 41, such that the measurements by the temperature sensor 42 are taken simultaneously as the heat production by the heating wire 41 is started.

The heat flow module 4 further comprises an electronic control unit 5, which is provided in the middle of the heat flow module 4 such that it extends through the inner sleeve 44. The electronic control unit 5 is arranged to control the heat provided by the heating wire 41 and retrieve the measurement results from the temperature sensor 42 as the heat dissipation through the soil is measured.

An insulation ring 6 is provided at both ends of the heat flow module 4. The insulation ring 6 is used to help prevent heat loss into the rod above and below the inner sleeve 44, in an axial direction of the heat flow cone penetrometer system 1. In addition, the insulation ring 6 is arranged such that the inner sleeve 44 and the dissipation sleeve 43 are supported by the insulation ring 6. As a result, an insulating air gap is defined between the inner sleeve 44 and an inner core of the heat flow cone penetrometer system 1 to prevent heat loss from the core of the heat flow cone penetrometer system 1 in a radial direction.

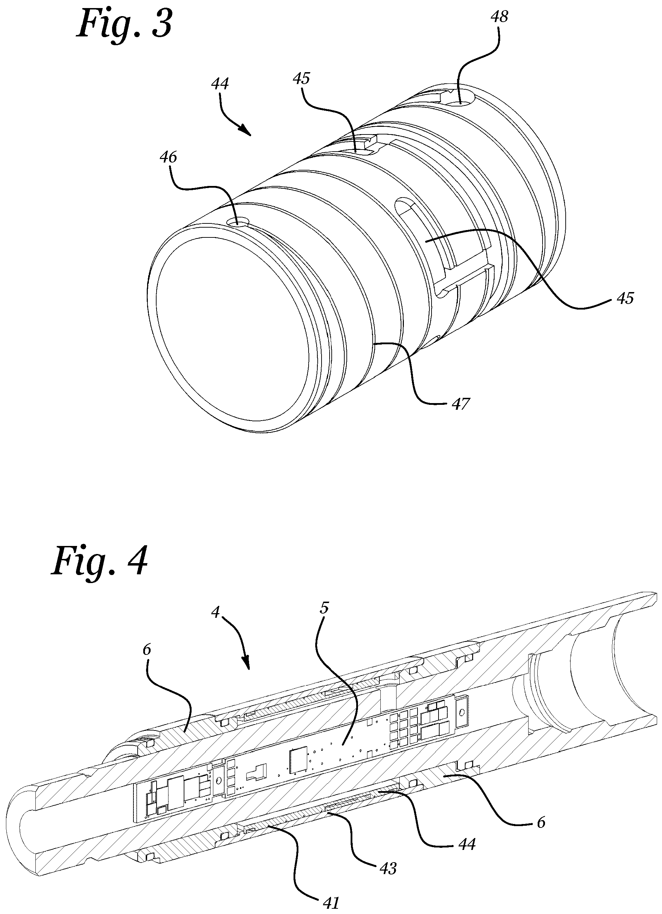

Now referring to FIG. 3, a three-dimensional view of the inner sleeve 44 of a heat flow module 4 in the heat flow cone penetrometer system 1 according to an example is shown. The inner sleeve 44 of the shown example comprises a set of two offset spiral grooves 47, which are arranged to receive a heating wire 41. The inner sleeve further comprises a plurality of slots 45, arranged to receive temperature sensors 42. These temperature sensors 42 are arranged to measure the change in temperature of the heat flow module 4 to determine the heat dissipation through a target soil.

The inner sleeve 44 of the shown example further comprises a hole 46, which extends through the inner sleeve 44 from an outer surface of the inner sleeve 44 to an inner surface of the inner sleeve 44. The hole 46 is arranged to receive the heating wire 41, which may protrude through the hole 46 and extend through the inner sleeve 44 such that the heating wire 41 may be controlled and provided with power from within the inner sleeve 44 where the electronic control unit 5 is provided.

The inner sleeve 44 further comprises a temperature sensor cable hole 46, which is provided separately from the through-hole 46 for the heating wire 41. The temperature sensor cable hole 46 is provided such that a cable extending from the temperature sensor 42 may extend through the inner sleeve 44 in a similar manner as the heating wire 41, but without being provided in the same hole. The inner sleeves 44 of FIG. 2 and FIG. 4 may have the same features of the inner sleeve 44 depicted in FIG. 3 and described above.

Now referring to FIG. 4, a three-dimensional cross-sectional view of a section of the heat flow cone penetrometer system 1, such as that depicted in FIG. 1 and FIG. 2, according to an example is shown. The section illustrates a heat flow module 4 of the heat flow cone penetrometer system 1. The heat flow module 4 of the shown example comprises an inner sleeve 44, such as the inner sleeve depicted in FIG. 3, and a dissipation sleeve 43 provided around the inner sleeve 44. The inner sleeve 44 comprises grooves 47, in which a heating wire 41 is contained, such that the heating wire 41 turns around the inner sleeve 44. A plurality of temperature sensors 42 are provided in slots provided on an outer surface of the inner sleeve.

The heat flow cone penetrometer system 1 comprises an electronic control unit 5 provided in the heat flow module 4. In the shown example, a single electronic control unit 5 is provided in a single heat flow module 4. Similar heat flow modules 4 may also comprise an electronic control unit 5. Alternatively, one electronic control unit 5 may be utilized to control a plurality of heat flow modules 4.

Two insulating rings 6 are provided on either side of the heat flow module 4, such that the insulation rings 6 support the dissipation sleeve 43 and the inner sleeve 44. The insulation rings 6 are provided such that they reduce the internal heat flow in an axial and radial direction of the heat flow cone penetrometer system 1, improving the data acquisition quality.

### Heat flow needle probe

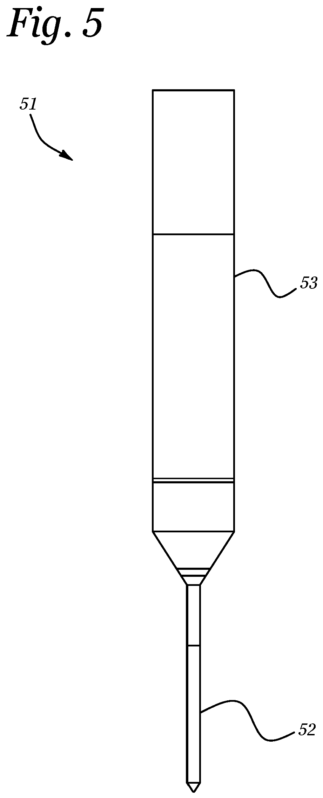

Now referring to FIG. 5, an example heat flow needle probe 51 is depicted. A thin, elongated heat flow needle 52 is provided at one end of the probe 51 and a pushrod 53 is provided at the other. The heat flow needle 52 consists of both a heating element and a temperature sensor and is actively heated and is thus not dependent on friction-induced heating. When the probe is inserted into the soil, the probe is heated, and the temperature of the probe is recorded. Using the temperature data of the probe, the thermal conductivity of the soil is found. After heating, an additional cooling phase can be added to the test to improve the accuracy of the test. During this cooling phase, the temperature dissipation of the probe is measured.

A needle is used since it defines an aspect ratio of length with respect to its diameter which allows the thermal conductivity to be modelled under the assumption that the needle is defined as an infinite line heat source model, which has an analytical solution to energy dissipation that can be resolved with a relatively short measurement time.

The diameter of the heat flow needle 52 is less than that of the heat flow cone penetrometer system 1. Following Equation 1, a shorter measurement window would be required using the needle probe in order to provide an accurate result for thermal conductivity. Further, it has a sufficiently small diameter-length ratio such that it adheres to an infinite line heat source model and errors in interpretation of the probe due to it not adhering to the infinite line heat source model do not arise. For example, the heat flow needle 52 may have a 3 mm diameter, a length of 200 mm, and D/L-ratio of 0.015. The heat flow cone penetrometer system 1 may have a diameter of 44 mm, a length of 94 mm, and a D/L-ratio of 0.468

### Heat conduction equation and calculation method for heat flow needle probe

In this section, the equations that are used for determining the heat flow through soil are described. The one-dimensional axisymmetric heat conduction equation (Equation 8) forms the basis for the method of obtaining a value for thermal conductivity using a heat flow needle probe 51.

Heat flows through soil due to three different mechanisms: conduction, convection, and radiation. The effect of radiation in soils is negligible ('Conduction of Heat in Solids' Carslaw & Jaeger, 1959). Convection in soil only plays an important role if there are groundwater flow conditions ('Thermal Cone Test to Determine Soil Thermal Properties' Akrouch et al., 2016). This makes conduction the only important mechanism to consider in soils without groundwater flow conditions.

Fourier's law (Equation 2) is commonly used to calculate heat flow and is as follows: $q = - k ⋅ ∇ T$ (2) where
q is the vector of heat flow [W/m² ]
k is the thermal conductivity [W/(mK)]
VT is the gradient of the temperature [K/m]

The heat balance of a soil element is described by the following equation (Carslaw & Jaeger, 1959): $C \frac{δ T}{δ t} + ∇ q = 0$ (3) where
C is the volumetric heat capacity [J/(m³ K)]
T is the temperature [K]
t is the time [s]

When the properties of the soil are homogeneous and the thermal conductivity does not depend on the heat of the soil, Equations 2 and 3 are combined to form the general heat conduction equation (Carslaw & Jaeger, 1959). $\frac{1}{D} \frac{δ T}{δ t} = {∇}^{2} T$ (4) with $D = \frac{k}{C}$ (5) where
D is the thermal diffusivity [m² /s]

Equation 4 is written in Cartesian coordinates as (Carslaw & Jaeger, 1959): $\frac{1}{D} \frac{δ T}{δ t} = \frac{{δ}^{2} T}{δ {x}^{2}} + \frac{{δ}^{2} T}{δ {y}^{2}} + \frac{{δ}^{2} T}{δ {z}^{2}}$ (6)

Equation 4 can also be written in cylindrical coordinates as (Carslaw & Jaeger, 1959): $\frac{1}{D} \frac{δ T}{δ t} = \frac{{δ}^{2} T}{δ {r}^{2}} + \frac{1}{r} \frac{δ T}{δ r} + \frac{1}{{r}^{2}} \frac{{δ}^{2} T}{{δθ}^{2}} + \frac{{δ}^{2} T}{δ {z}^{2}}$ (7)

If the heat flow is assumed to be constant in the z and θ direction, Equation 7 can be written as a one-dimensional axisymmetric equation where the heat conduction is only dependent on r (Carslaw & Jaeger, 1959): $\frac{1}{D} \frac{δ T}{δ t} = \frac{{δ}^{2} T}{δ {r}^{2}} + \frac{1}{r} \frac{δ T}{δ r}$ (8)

The thermal needle probe method interprets the thermal conductivity of soils by applying a transient heat source to the soil and monitoring the temperature change. When using a thermal needle probe, the thermal conductivity is found from a solution for the one-dimensional axisymmetric heat conduction (Equation 8). This solution assumes that there is an infinite line heat source with a constant heat input at r = 0. As the length of the probe is significantly larger than the diameter of the probe, in the case of a needle probe, this solution is valid (Shiozawa & Campbell, 1990). The solution for an infinite line heat source is outlined in ASTM D5334 and described by the following equation (Carslaw & Jaeger, 1959): $T - {T}_{0} = - \frac{Q}{4 π k} Ei \left(-\frac{{Cr}^{2}}{4 kt}\right)$ (9) where
*T*₀ is the initial temperature [K]
*T* is the temperature [K]
*Q* is the heat release per unit of length [W/m]
where *Ei* is the is the exponential integral function, defined as: $- Ei \left(-x\right) = {\int }_{x}^{∞} \frac{exp \left(-u\right)}{u} du$ (10)

When the term inside the exponential integral is small, as is the case for the small diameter of a thermal needle probe, *Ei* is estimated with the following series: $Ei \left(-x\right) = γ + ln \left(x\right) - x + \frac{1}{4} {x}^{2} + ⋯$ (11) where
γ = 0.577216 i.e. Euler's constant

For large values of *t* ( $t > \frac{5 {Cr}^{2}}{k}$), the *x* term in Equation 11 is small and the terms after In(x) are neglected in this equation. Equation 9 is in this case rewritten to (Carslaw & Jaeger, 1959): $T - {T}_{0} = \frac{Q}{4 π k} \left(-γ-ln\left(\frac{{Cr}^{2}}{4 kt}\right)\right)$ (12)

This is further rewritten to (Carslaw & Jaeger, 1959): $T - {T}_{0} = \frac{Q}{4 π k} \left(-γ-ln\left(\frac{{Cr}^{2}}{4 k}\right)+ln\left(t\right)\right)$ (13)

Note that -y and $ln \left(\frac{{Cr}^{2}}{4 k}\right)$ are constants that are independent of time. When taking the gradient of *T - T*₀ against ln(*t*), these parts are zero and the thermal conductivity is found with the following equation (ASTM D5334, 2022): $k = {f}_{NP} \frac{Q}{4 π S}$ (14) where
*f_{NP}* is a newly introduced calibration factor for the thermal needle probe and information on deriving this is shown in ASTM D5334 (2022) 'Standard Test Method for Determination of Thermal Conductivity of Soil and Rock by Thermal Needle Probe Procedure'.
S is the gradient of the measured temperature against In(t), occurring at a long timescale.

In the above manner, thermal conductivity of soil can be found using the thermal needle probe method, which enables use of a one-dimensional analytical solution to energy dissipation. However, for the aforementioned reasons, this method does not work for probes with a larger radius and a larger diameter-length ratio, such as the heat flow cone penetrometer system 1 described herein. As such, another method is needed as shall now be explained.

### Method of determining subsurface thermal properties of target soil using a heat flow cone penetrometer

In order to be able to determine thermal conductivity of soil using a measurement probe of larger diameter and larger diameter-length ratio than a heat flow needle probe 51, such as the heat flow cone penetrometer system 1, the following method is provided. The method is described in relation to the heat flow cone penetrometer system 1, but it will be understood that other heat flow penetrometers can be used. The method could also be used for needle probes, such as heat flow needle probe 51, or other shaped probes, such as a penetrometer system with a square heating module instead of a cylindrical or cone shape.

As outlined above, an analytical solution to energy dissipation is not a viable option for probes of larger diameter, and larger diameter-length ratio than heat flow needle probe. Another problem arising with larger diameter probes is that it, in practice, may not consist of a homogeneous material. As a result, a new numerical method is required to capture influences for such large diameter probe designs. Heat flow through a homogeneous material can be described by analytical solutions, whereas a non-homogeneous probe does not adhere to such solutions. Numerical simulations are required to take into account effects of electronic components, air gaps and seals, thermal insulation layers, and several thermal conductive components on measurements with the large diameter probe. As a result, an analytical solution to energy dissipation is also not a viable option probes consisting of several different material combinations, such as a heat flow cone penetrometer system 1.

In light of these issues, a numeric forward model, such as finite element method (FEM), numerical forward solvers and numeric simulation tools, is constructed to accurately predict the thermal response of the heat flow cone penetrometer system 1, with the thermal parameters of the soil (such as thermal conductivity and volumetric heat capacity) as an input. This numeric forward model can be inverted in order to obtain predicted values for the thermal parameters of target soil using the measured thermal response of the heat flow cone penetrometer system 1.

An example implementation of the numeric forward model is a numerical solution made with finite element modelling software, simulating the heat flow cone penetrometer system 1 thermal response with 2D or 3D fine element analysis (FEA) in, such as COMSOL, ABAQUS, ANSYS or any other commercial or non-commercial numerical forward solver. For example, the model can be a 2D axisymmetric problem with heat conduction as the only heat transfer mechanism. The model can be based on the geometry or geometrical characteristic(s) of the measurement probe used, in this instance the geometry of the aforementioned heat flow cone penetrometer system 1. A simplified geometry (for example an axisymmetric and/or a solid and homogeneous probe) of the heat flow cone penetrometer system 1 can be used to reduce model runtime. Alternatively, the exact dimensions of the heat flow cone penetrometer system 1 may be used. The finite element method can predict the conductive heat transfer occurring between the measurement probe and the soil by solving the general heat conduction differential equation (Equation 4).

Geometrical characteristics of the measurement probe used, in this instance the geometry of the aforementioned heat flow cone penetrometer system 1, upon which the model can be based include one or more of: length of heating element 41; diameter (or width when not cylindrical) of the heating element; size, shape and position of an air gap (e.g. the aforementioned insulating air gap is defined between the inner sleeve 44 and an inner core or between the inner sleeve 44 and the dissipation sleeve 43); shape and position of any seals; geometry of heating element 41; wall thickness of insulation element (e.g. the aforementioned insulation rings 6); diameter and wall thickness of inner body of heat flow module (e.g. a section arranged to translate a pressure force on the penetrometer); and size of cavity inside the heat flow module containing the electronic components.

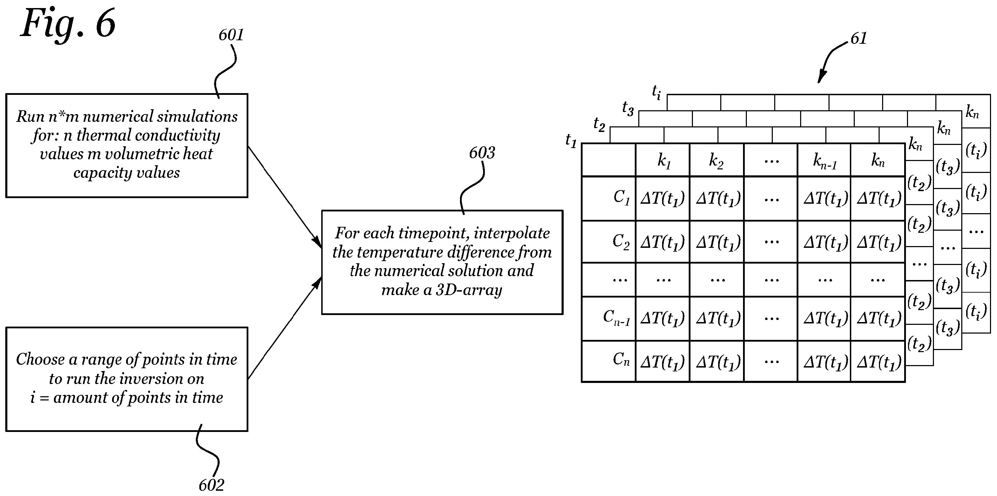

Next to geometry, the thermal propertiesor characteristics of these elements or the measurement probe used are also important for formation of the solution space. For example, the thermal conductivity of the insulation material and the components of the penetrometer such as the inner sleeve and the dissipation sleeve. Example thermal characteristics include one or more of: shape/geometry, weight, density, thermal conductivity, volumetric heat capacity of one or more of the constituent materials/elements of the the measurement probe used. Different geometry and thermal properties of the heat flow cone penetrometer system 1 will lead to a different thermal response and hence a different set of values on the solution space 61 (as depicted in FIG. 6 and discussed in detail below). in this manner a solution space 61 can be unique to a specific heat flow cone penetrometer design.

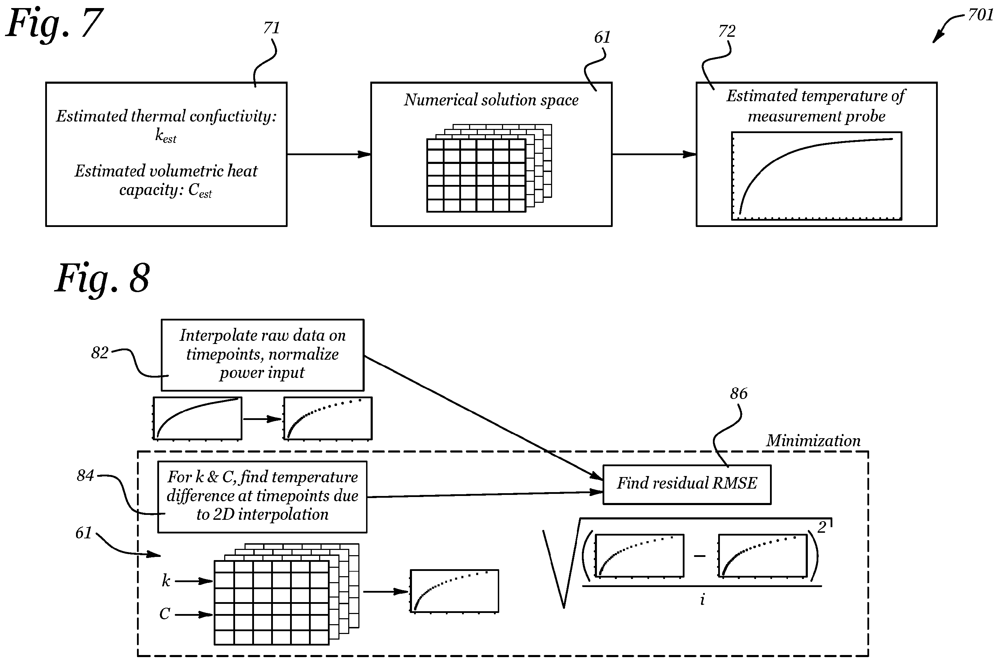

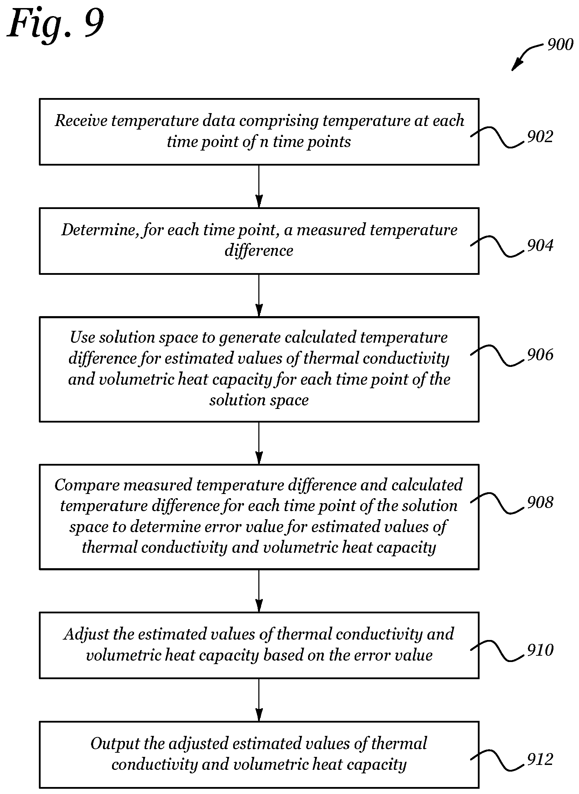

Running a numeric forward model, such as the model described above, is time consuming and increases the overall time needed to determine soil properties using the heat flow cone penetrometer system 1. In order to address this issue, a numeric forward model is not run during the process in which the measured thermal response of the heat flow cone penetrometer system 1 is used to determine thermal parameters of target soil (as outlined in FIG. 9 and the corresponding description). Instead, a solution space 61 (as depicted in FIG. 6) is created in advance that stores results generated by the numeric forward model of a predicted thermal response of the measurement probe for specific thermal conductivity and volumetric heat capacity values. This solution space 61 is then interpolated for an estimated thermal conductivity and volumetric heat capacity value to extract a numeric forward model result (i.e., a temperature difference versus time curve belonging to the best guess thermal values). Then this numeric forward model result is compared with the actual measured temperature difference versus time curve obtained by the heat flow cone penetrometer system 1 test in soil (i.e., raw measured data); a minimization routine (iterative calculation) is used to find the best match between the raw measurement data and the stored numeric forward model results. The solution space interpolation and minimization routine are hereafter defined as the inversion method. A schematic overview of some steps of the inversion method is shown in FIG. 7 and FIG. 8.

### Constructing a solution space

Referring initially to FIG. 6, a schematic overview of the process of constructing a solution space 61 for use in the inversion method shown in FIG. 7, FIG. 8 and FIG. 9 is depicted. In order to run the inversion method, a solution space 61 is created that stores a wide range of numeric forward model simulations for a particular measurement probe, in this instance the heat flow cone penetrometer system 1. This solution space 61 can then subsequently be used to perform an interpolation to find the temperature difference over time for input thermal conductivity and volumetric heat capacity values.

There are three factors that influence the size of the solution space 61:
- The range of thermal conductivity values in the solution space 61 [W/(mK)]
- The range of volumetric heat capacity values in the solution space 61 [J/(m³K)]
- The range of time points on which the inversion method is performed [s]

The size and values of these ranges can be chosen to suit a particular need. In general, a larger number of values leads to a more accurate match with the numeric forward model when using the inversion method. However, increasing the number of values also extends the amount of time needed to both construct the solution space 61 and perform the inversion method to determine the thermal properties of soil.

After the ranges of values are determined, the numeric forward model is run for all different thermal conductivity and volumetric heat capacity values in the ranges (see step 601 in FIG. 6). As an example, for 20 different values of thermal conductivity and 20 different values of volumetric heat capacity, the numeric forward model runs 400 times to cover all combinations of those values. The output of each run of the numeric forward model is a predicted thermal response of the specific measurement probe to which the numeric forward model relates, in this instance the heat flow cone penetrometer system 1. The temperate response of the heat flow cone penetrometer system 1 takes the form of a plot of temperature difference over time, where temperature difference is the difference between an initial temperature of the probe and a temperature at each time point thereafter. For example, the initial temperature of the probe could be the temperature of the probe after it has been inserted into target soil at a desired depth and before it has been heated up. The temperature at each time point as it is heated up can then be recorded.

As outlined above, alongside the range of thermal conductivity values and the range of volumetric heat capacity values, the range of points in time (*t*₁ *to tᵢ*) on which the inversion method is to be performed is determined (see step 602 in FIG. 6). For each numeric forward model run, the temperature difference Δ*T* may optionally be interpolated, for example using a linear interpolation between two points, for each determined time point (see step 603 in FIG. 6), such that a value for temperature difference Δ*T* is obtained for each time point (*t*₁ *to tᵢ*) of the solution space. Each temperature difference Δ*T* is stored in the solution space 61 as a three-dimensional array with the thermal conductivity values (*k*₁ *to kₙ* in FIG. 6) on the x-axis, the volumetric heat capacity values (*C*₁ *to Cₙ* in FIG. 6) on the y-axis, and the different time points (*t*₁ *to tᵢ* in FIG. 6) on the z-axis.

As the numeric forward model used to generate the solution space 61 is dependent on the geometry of a particular measurement probe, in this instance the heat flow cone penetrometer system 1, a different solution space can be generated using a different numeric forward model where a measurement probe of a different geometry is to be used.

Once the solution space 61 has been generated, it can be used in the inversion method to obtain predicted values for thermal conductivity and volumetric heat capacity based on a measured thermal response of a measurement probe, in this instance the heat flow cone penetrometer system 1. The inversion method will now be described.

### Inversion method

Elements of the inversion method are depicted in FIG. 7 and FIG. 8, which will briefly be described below. The full method is depicted in FIG. 9 and also described below.

FIG. 7 depicts a schematic overview of a part of the inversion method, as will be explained in more detail below. The process 701 depicts input estimated values of thermal conductivity, k, and volumetric heat capacity, *C,* 71. These values are used in conjunction with the solution space 61 to generate a calculated temperature difference, Δ*T̂,* for the estimated values of thermal conductivity, *k,* and volumetric heat capacity, *C,* for each of a predetermined series of time points, *tᵢ*, of the solution space 61. The generated temperature differences, Δ*T̂,* reflect an estimated temperature response 72 of a measurement probe, in this instance the thermal response of the heat flow cone penetrometer system 1 in soil having the estimated values of thermal conductivity, *k,* and volumetric heat capacity, *C.*

FIG.8 depicts a schematic overview of a different part the inversion method to FIG. 8. The optional steps of interpolating and normalising 82 the measured temperature data output by the heat flow cone penetrometer system 1 are depicted, as is the interpolation 84 of the of the solution space 61 to find a calculated temperature difference, Δ*T̂,* for the estimated values of thermal conductivity, *k,* and volumetric heat capacity, *C,* for each of a predetermined series of time points, *tᵢ*, of the solution space 61. Finally, the adjusting of the estimated values of thermal conductivity, k, and volumetric heat capacity, C, using an optional root mean square error (RMSE) and minimization 86 is depicted. The inversion method, shown in overview in Figures 7 and 8, will now be described in greater detail.

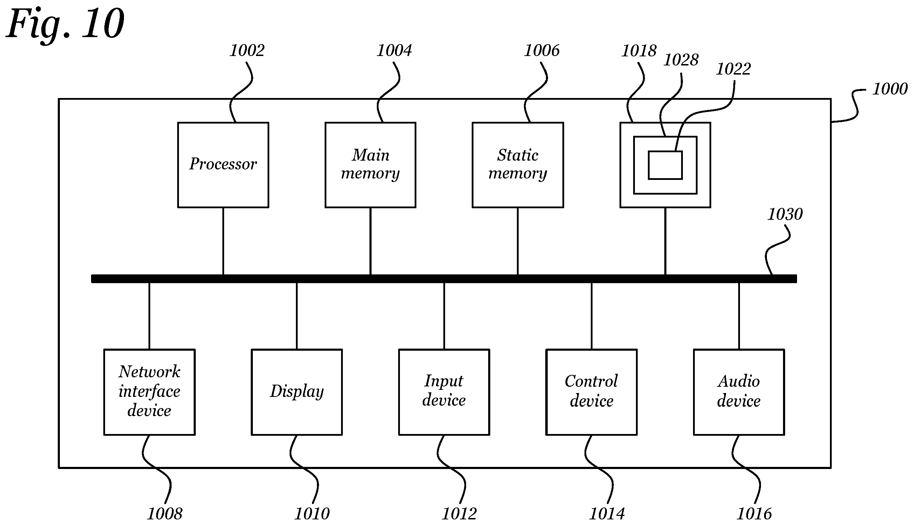

FIG. 9 depicts a flowchart of a method 900, implemented on a computer, of determining subsurface thermal conductivity of target soil. The method 900 may, for example, be implemented at one or more processors of the apparatus 1000 shown in FIG. 10. In particular, the method 900 may be implemented in the form of an application comprising instructions stored on a transitory or non-transitory computer-readable medium (as described further below), or a computer program, wherein the instructions are executable by the one or more processors to cause the one or more processors to implement the method 900. The method is described in relation to the heat flow cone penetrometer system 1, but it will be understood that other heat flow penetrometers can be used. The method could also be used for needle probes, such as heat flow needle probe 51, or other shaped probes, such as a penetrometer system with a square heating module instead of a cylindrical or cone shape. As can be appreciated in light of the disclosure, the order of steps within the method 900 is not limited to the sequential execution as illustrated in FIG. 9 but may be performed in one or more varying orders as applicable and in accordance with the present disclosure.

At step 902, temperature data comprising a temperature, *T,* at each time point *tⱼ* of *n* time points is received from the heat flow penetrometer 1 that is surrounded by target soil.

At step 904, a measured temperature difference, Δ*T,* is determined for each time point *tⱼ.* In an example implementation, the temperature difference is obtained by subtracting an initial temperature, *T*₀ of the heat flow penetrometer 1 from the temperature, *T,* at each time point, *tⱼ.* In another example implementation, the temperature difference is obtained with respect to the temperature of the previous time point *t*_{*j*-1}.

At step 906, based on estimated values of thermal conductivity, *k*, and volumetric heat capacity, *C*, of the target soil, the solution space 61 is used to generate a calculated temperature difference, Δ*T̂,* for the estimated values of thermal conductivity, *k*, and volumetric heat capacity, *C*, for each of a predetermined series of time points, *tᵢ*, of the solution space. The solution space 61 comprises: a plurality of values of thermal conductivity, *k*; a plurality of values of volumetric heat capacity, *C*; and pre-computed values of temperature difference, Δ*T,* for each combination of values of thermal conductivity, *k,* and volumetric heat capacity, *C,* and for each of the predetermined series of time points, *tᵢ*, of the solution space 61. It will be understood that step 906 may be carried out before, contemporaneously with, or after step 904.

At step 908, the measured temperature difference Δ*T* obtained at block 904 and the calculated temperature difference, Δ*T̂,* obtained at block 906 for each time point, *tᵢ*, of the solution space are compared to determine an error value for the estimated values of thermal conductivity, *k,* and volumetric heat capacity, *C.*

At step 910, the estimated values of thermal conductivity, k, and volumetric heat capacity, C, of the target soil are adjusted based on the error value.

At step 912, the adjusted estimated values of thermal conductivity, *k,* and volumetric heat capacity, *C,* are output. This output provides an estimated value of thermal conductivity, k, and volumetric heat capacity, C, for the target soil into which the heat flow penetrometer 1 is inserted.

Considering the above steps in greater detail, in order for the temperature data to be received at step 902, the heat flow penetrometer 1 is inserted into the target soil for which thermal conductivity and volumetric heat capacity are to be determined. The heat flow penetrometer 1 starts at an initial temperature *T*₀ and is then actively heated up. The temperature change over time is then recorded. Optionally or alternatively, the heat flow penetrometer 1 can be heated to a desired initial temperature *T*₀ and subsequently be allowed to cool as heat dissipates into the target soil and temperature over time is recorded as this happens. The measurement window could be either the heating up, cooling down, or heating up and cooling down of the heat flow penetrometer 1. It could also be an initial cooling down of the heat flow penetrometer 1 followed by a heating of the heat flow penetrometer 1 or any combination of heating and cooling of the heat flow penetrometer 1, for example, a sequence of heating and cooling or cooling and heating. The measured temperature data obtained over the measurement window is then sent to the computer carrying out the method 900. This measured temperature data consists of a column of time points *t*₁ *to tⱼ* and a corresponding column of temperature values *T*₁ *to Tⱼ* measured per time point.

Where the measurement window covers temperature data recorded while the heat flow penetrometer 1 is cooling down, a problem could arise as the power input may not be known. In that case the solution space 61 could be expanded to cover several power levels. In other words, the solution space 61 could contain data corresponding to temperature-time curves for a range of thermal conductivities, *k,* and a range of volumetric heat capacities, *C,* and range of power levels.

The measured temperature differences Δ*T* determined at step 904 may be obtained by subtracting the initial temperature, *T*₀, from the temperature, *Tₜⱼ* for each time point *tⱼ.* In other words, Δ*Tₜⱼ = Tₜⱼ - T*₀*.* Alternatively, the measured temperature differences Δ*T* determined at step 904 may be obtained by subtracting a previous temperature, *T*_{*tj-*1}*,* from the temperature, *Tₜⱼ,* for each time point *tⱼ.* In other words, Δ*Tₜⱼ* = *Tₜⱼ - T*_{*tj-*1}*.*

At this point, an optional step of normalising the temperature difference Δ*T* at each time point *tⱼ* 82 (shown in FIG. 8) can be performed to match the power level for which the temperature was generated by the heat flow penetrometer 1 to the power level used in the numeric forward model to generate the solution space 61. An advantage of including this normalization step is that the power level of the heat flow penetrometer 1 does not need to be matched to that used when generating the solution space. In this way, the influence of power level is removed from the inversion method. The following equation can be used for the normalisation: $Δ {T}_{norm} = ΔT ∗ \frac{{Q}_{ref}}{{Q}_{data}}$ (15) where
Δ*Tₙₒᵣₘ* is the normalized temperature difference [K]
ΔT is the measured temperature difference obtained from the heat flow penetrometer 1 temperature data [K]
*Q_{ref}* is a reference the power level used for normalisation, which in this case is equal to the power level for which the solution space is generated [W]
*Q_{data}*is the power level for which the heat flow penetrometer 1 temperature data is generated [W]

After the measured temperature differences Δ*T* at each time point *tⱼ* determined at step 904 are normalized for power level, a further optional step can be applied in which the measured temperature difference, Δ*T,* is matched to the solution space 61 based on the predetermined series of time points, *tᵢ* of the solution space using an interpolation method, for example linear interpolation 82 (shown in FIG. 8). The result is a set of temperature differences Δ*T* corresponding to the measured temperature data for each of the time points, *tᵢ* of the solution space 61. Where the column of time points *t*₁ *to tⱼ* for which the temperature data of the heat flow penetrometer 1 is obtained does not match the time points (*t*₁ *to tᵢ* in FIG. 6) of the solution space 61, the interpolation performed here ensures that they do. As such, it allows for the temperature data of the heat flow penetrometer 1 to be produced with any column of time points *t*₁ *to tⱼ* that do not necessarily need to match those of the solution space 61.

Estimated values of thermal conductivity, k, and volumetric heat capacity, *C*, of the target soil are required at step 906. These can be estimated by any suitable means. As an example, 'CPT correlations for thermal properties of soils' by Vardon and Peuchen (2020) provides the equations for predicting a value for the thermal conductivity of soil and for predicting a value for the volumetric heat capacity of soil.

Once estimated values of thermal conductivity, *k,* and volumetric heat capacity, *C*, are obtained, in step 906 the solution space 61 is used to generate a calculated temperature difference, Δ*T̂,* for the estimated values of thermal conductivity, *k,* and volumetric heat capacity, *C,* for each of a predetermined series of time points, *tᵢ*, of the solution space 61. This could be for every time point, *tᵢ*, of the solution space 61 or it could be for a subset of the total number of time points, *tᵢ*, of the solution space 61. This process is depicted in process 701 of FIG. 7 and item 84 in FIG. 8.

As described above, for each time point, *tᵢ*, the solution space is divided into a 2D grid of temperature differences, with the thermal conductivity on the x-axis and the volumetric heat capacity on the y-axis (see FIG. 6). In order to find a calculated temperature difference, Δ*T̂,* for the estimated thermal conductivity and volumetric heat capacity values 71 at a specific time point, *tᵢ*, this grid is interpolated with a 2D interpolator, for example a 2D linear interpolator, to find the calculated temperature difference Δ*T* of the specific time point, *tᵢ*, for the estimated thermal conductivity and volumetric heat capacity values 71. This 2D interpolation is performed each of the predetermined series of time points, *tᵢ*, of the solution space 61, which could be every time point, *tᵢ*, of the solution space 61 or it could be a subset of time points, *tᵢ*, of the solution space 61.

As described above, step 908 involves a comparison between measured and calculated values of temperature difference. In particular, once a calculated temperature difference, Δ*T̂,* for the estimated thermal conductivity and volumetric heat capacity values 71 has been obtained for each of the predetermined series of time points, *tᵢ*, of the solution space 61, the calculated temperature differences, Δ*T̂,* are compared with the corresponding measured temperature differences, Δ*T ,* for each time point, *tᵢ*, of the solution space 61 at step 908 to determine an error value for the estimated values of thermal conductivity, *k,* and volumetric heat capacity, *C.* An example optional implementation of such a comparison is by determining the root mean square error (*RMSE*) for each calculated temperature differences, Δ*T̂,* compared with the corresponding measured temperature differences, Δ*T ,* for each time point, *tᵢ*, of the solution space 61. The following equation can be used for this purpose: $RMSE = \sqrt{{\sum }_{i = 1}^{n} \frac{{\left(Δ\hat{T}\left({t}_{i}\right)-ΔT\left({t}_{i}\right)\right)}^{2}}{n}}$ (16) where
RMSE is the root mean square error
Δ*T̂*(*tᵢ*) is the calculated temperature difference found from the solution space at timepoint *tᵢ* [K]
Δ*T*(*tᵢ*) is the measured temperature difference at timepoint *tᵢ*, optionally corrected for the power level [K]
n is the total number of timepoints for which the inversion is performed

The smaller the value of the RMSE is, the better the match is between the measured temperature differences, Δ*T* , and the calculated temperature differences, Δ*T̂,* generated with the solution space 61. For example, a RMSE of zero means that the temperature difference for each time point, *tᵢ*, is identical for the measured temperature differences, Δ*T* , and the calculated temperature differences, Δ*T̂,* generated with the solution space 61.

An alternative method to determining the RMSE is to determine mean square error (MSE) or mean absolute error (MAE).

Once an error value for the estimated values of thermal conductivity, k, and volumetric heat capacity, C, has been determined at step 908, the estimated values of thermal conductivity, *k,* and volumetric heat capacity, *C,* of the target soil based on the error value are adjusted at step 910. A lower value of the RMSE means a better match between the measured temperature differences, Δ*T ,* and the calculated temperature differences, Δ*T̂,.* For the lowest value of the RMSE, the best match between the two is found. As the calculated temperature differences, Δ*T̂,* is generated for a specific estimated volumetric heat capacity and estimated thermal conductivity value, these two values can be adjusted in order to minimize the RMSE. An example optional implementation of this adjustment is via the use of a minimization solver.

Using this minimization solver, the estimated thermal conductivity and volumetric heat capacity values are found for which the dataspace has the best match with the measured temperature differences, Δ*T.* The Nelder-Mead method may be used as the minimization method, which does not require the calculation of function derivatives and can handle boundaries. The boundaries of the minimization are set to the minimum and maximum values of thermal conductivity and volumetric heat capacity found in the solution space 61. A first estimation of the thermal conductivity and volumetric heat capacity is provided as initial value of the minimization. This final output of the minimization solver is the aforementioned best match between the measured temperature differences, Δ*T ,* and the calculated temperature differences, Δ*T̂,* from which the best match estimations for thermal conductivity, *k,* and volumetric heat capacity, *C,* of the target soil can be found.

An alternative example of a minimization solver is the conjugate gradient method. The conjugate gradient method is an algorithm for the numerical solution of particular systems of linear equations, namely those whose matrix is positive-definite. The conjugate gradient method is often implemented as an iterative algorithm, applicable to sparse systems that are too large to be handled by a direct implementation or other direct methods such as the Cholesky decomposition. Large sparse systems often arise when numerically solving partial differential equations or optimization problems. The conjugate gradient method can also be used to solve unconstrained optimization problems such as energy minimization.

To further improve the inversion method, the inversion method can, optionally, be regularized based on the estimated volumetric heat capacity. By regularizing the inversion method, an error function is added to the minimization solver which penalises volumetric heat capacities found by the inversion method deviating further away from the estimated volumetric heat capacity. The further the solution is from the expected estimated volumetric heat capacity, the higher the penalty of the function is. A quadratic loss function may be added to the equation that is minimized (Equation 16) in order to achieve this. The quadratic loss function is symmetric in nature which means that no difference in penalty is made for over or underestimation of the estimated volumetric heat capacity. The function that is minimized then contains two parts:
- The RMSE function that gives the error between the raw data and the parametric space for specific thermal properties (Equation 16).
- The quadratic error function that penalizes the inversion method when a volumetric heat capacity value is found that differs from the estimated volumetric heat capacity.

In order to assure that these two parts have the same relative influence on the minimization solver, independent of the power level and thermal properties of the test, further normalizations can performed. In one example, the RMSE is divided by the total temperature difference of the measured temperature data received from the heat flow penetrometer 1 to form the normalized root mean square error (NRMSE) as follows: $NRMSE = \frac{RMSE}{max \left(ΔT\left({t}_{i}\right)\right) - min \left(ΔT\left({t}_{i}\right)\right)}$ (17) where
*NRMSE* is the normalized root mean square error
*RMSE* is the root mean square error
Δ*T*(*tᵢ*) is the temperature difference of the measured temperature data found for the solution space time points [K]

All thermal conductivity and volumetric heat capacity values used in the minimization solver are also normalized beforehand, keeping all values within a range from 0 to 1. The minimization function combining the NRMSE and the quadratic penalty function is as follows: $f \left({k}_{norm}, {C}_{norm}\right) = NRMSE \left({k}_{norm}, {C}_{norm}\right) + {W}_{r} ⋅ {\left({C}_{exp ; norm}-{C}_{norm}\right)}^{2}$ (18) ${k}_{norm} = \frac{k - min \left({k}_{data}\right)}{max \left({k}_{data}\right) - min \left({k}_{data}\right)}$ (19) ${C}_{norm} = \frac{C - min \left({C}_{data}\right)}{max \left({C}_{data}\right) - min \left({C}_{data}\right)}$ (20) ${C}_{norm ; exp} = \frac{{C}_{exp} - min \left({C}_{data}\right)}{max \left({C}_{data}\right) - min \left({C}_{data}\right)}$ (21) where
*f*() is the function that is minimized
*Wᵣ* is the regularization constant, a value from 0 to 1
*k_{data}* is the range of thermal conductivity values in the solution space 61 [W/(mK)] solution space time points [K]
*Cₑₓₚ* is the expected or estimated value of the volumetric heat capacity, found by a CPT correlation or laboratory tests [J/(m³ K)]
*C_{data}* is the range of volumetric heat capacity values in the solution space 61 [J/(m³ K)]
*Cₙₒᵣₘ* is the normalised volumetric heat capacity value found in the solution space [-]
*kₙₒᵣₘ* is the normalised thermal conductivity value found in the solutions space [-]
*C*_{*norm*;*exp*} is the normalised expected volumetric heat capacity value [-]

The regularization constant, *Wᵣ*, determines the severity of the penalty added to the minimization. For a value of zero, no penalty for a deviation from the estimated volumetric heat capacity is added, so the inversion is the same as the regular inversion to find the thermal conductivity and the volumetric heat capacity. For a value of 1, the penalty of deviation is high, and the results tend to the results of an inversion method with a fixed volumetric heat capacity value.

At step 912, the adjusted estimated values of thermal conductivity, k, and volumetric heat capacity, C, of the target soil are output for further use.

FIG. 10 shows a block diagram of one implementation of a computing device 1000 within which a set of instructions, for causing the computing device to perform any one or more of the methodologies discussed herein, may be executed. In alternative implementations, the computing device may be connected (e.g., networked) to other machines in a Local Area Network (LAN), an intranet, an extranet, or the Internet. The computing device may operate in the capacity of a server or a client machine in a client-server network environment, or as a peer machine in a peer-to-peer (or distributed) network environment. The computing device may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 1000 includes a processor 1002, a main memory 1004 (e.g., read-only memory (ROM), flash memory, dynamic random access memory (DRAM) such as synchronous DRAM (SDRAM) or Rambus DRAM (RDRAM), etc.), a static memory 1006 (e.g., flash memory, static random access memory (SRAM), etc.), and a secondary memory (e.g., a data storage device 1018), which communicate with each other via a bus 1030.

Processor 1002 represents one or more general-purpose processors such as a microprocessor, central processing unit, or the like. More particularly, the processor 1002 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processor 1002 may also be one or more special-purpose processors such as an application specific integrated circuit (ASIC), a field programmable gate array (FPGA), a digital signal processor (DSP), network processor, or the like. Processor 1002 is configured to execute the processing logic (instructions 1022) for performing the operations and steps discussed herein.

The computing device 1000 may further include a network interface device 1008. The computing device 1000 also may include a video display unit 1010 (e.g., a liquid crystal display (LCD) or a cathode ray tube (CRT)), an alphanumeric input device 1012 (e.g., a keyboard or touchscreen), a cursor control device 1014 (e.g., a mouse or touchscreen), and an audio device 1016 (e.g., a speaker).

It will be apparent that some features of computer device 1000 shown in FIG. 10 may be absent. For example, one or more computing devices 1000 may have no need for display device 1010 (or any associated adapters). This may be the case, for example, for particular server-side computer apparatuses 1000 which are used only for their processing capabilities and do not need to display information to users. Similarly, user input device 1012 may not be required. In its simplest form, computer device 1000 comprises processor 1002 and memory 1004.

The data storage device 1018 may include one or more machine-readable storage media (or more specifically one or more non-transitory computer-readable storage media) 1028 on which is stored one or more sets of instructions 1022 embodying any one or more of the methodologies or functions described herein. The instructions 1022 may also reside, completely or at least partially, within the main memory 1004 and/or within the processor 1002 during execution thereof by the computer system 1000, the main memory 1004 and the processor 1002 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored

or otherwise embodied in a machine-readable medium or in a transmission medium).

Unless specifically stated otherwise, as apparent from the following discussion, it is appreciated that throughout the description, discussions utilizing terms such as " receiving", "determining", "comparing ", "enabling", "maintaining," "identifying," "generating," "adjusting," "outputting," or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described but can be practiced with modification and alteration within the scope of the appended claims. Accordingly, the specification and drawings are to be regarded in an illustrative sense rather than a restrictive sense.

The invention has been described by reference to certain aspects discussed above. It will be recognized that these aspects are susceptible to various modifications and alternative forms well known to those of skill in the art.

Further modifications in addition to those described above may be made to the structures and techniques described herein without departing from the scope of the invention. Accordingly, although specific aspects have been described, these are examples only and are not limiting upon the scope of the invention.

## Claims

1. A computer-implemented method (900) of determining subsurface thermal conductivity of target soil, the method comprising:
receiving (902), from a heat flow penetrometer that is surrounded by target soil, temperature data comprising a temperature, *T,* at each time point *tⱼ* of *n* time points;
determining (904), for each time point, *tⱼ,* a measured temperature difference, Δ*T*;
based on estimated values of thermal conductivity, *k,* and volumetric heat capacity, *C,* of the target soil, using a solution space (906) to generate a calculated temperature difference, Δ*T̂,* for the estimated values of thermal conductivity, *k*, and volumetric heat capacity, *C*, for each of a predetermined series of time points, *tᵢ*, of the solution space,
wherein the solution space comprises:
a plurality of values of thermal conductivity, *k*;
a plurality of values of volumetric heat capacity, *C*; and
pre-computed values of temperature difference, Δ*T,* for each combination of values of thermal conductivity, *k*, and volumetric heat capacity, *C*, and for each of the predetermined series of time points, *tᵢ*, of the solution space;
comparing (908) the measured temperature difference, Δ*T ,* and the calculated temperature difference, Δ*T̂,* for each time point, *tᵢ*, of the solution space to determine an error value for the estimated values of thermal conductivity, *k,* and volumetric heat capacity, *C;*
adjusting (910) the estimated values of thermal conductivity, *k,* and volumetric heat capacity, *C,* of the target soil based on the error value; and
outputting (912) the adjusted estimated values of thermal conductivity, k, and volumetric heat capacity, *C.*

2. The computer-implemented method of claim 1, further comprising the step of normalizing each measured temperature difference, Δ*T,* based on a reference power level, *Q_{ref},* and a power level at which the temperature data is generated, *Q_{data}*.

3. The computer-implemented method of either of claim 1 or claim 2, wherein the estimated values of thermal conductivity, *k,* and volumetric heat capacity, *C,* are adjusted using a minimization solver to minimize the error value.

4. The computer-implemented method of claim 3, wherein a regularization function is added to the minimization solver which provides a penalty when an adjusted estimated volumetric heat capacity, *C,* is generated that differs from a predetermined volumetric heat capacity, *C.*

5. The computer-implemented method of any preceding claim, wherein the pre-computed values of temperature difference, Δ*T,* are pre-computed using a numerical forward solver.

6. The computer-implemented method of any preceding claim, wherein the pre-computed values of temperature difference, Δ*T,* are pre-computed based on at least one geometrical characteristic of the heat flow penetrometer.

7. The computer-implemented method of any preceding claim, wherein the pre-computed values of temperature difference, Δ*T,* are pre-computed based on a thermal characteristic of the heat flow penetrometer.

8. The computer-implemented method of any preceding claim, further comprising the step of matching the measured temperature difference, Δ*T,* to the solution space based on the predetermined series of time points, *tᵢ* of the solution space and using an interpolation.

9. The computer-implemented method of any preceding claim, wherein the error value is determined by calculating the root mean square error of the measured temperature difference, Δ*T ,* and the calculated temperature difference, Δ*T̂,* for each time point, *tᵢ*, of the solution space.

10. The computer-implemented method of any preceding claim, wherein the solution space comprises a three-dimensional array with the plurality of values of thermal conductivity, *k,* on the x-axis, the plurality of values of volumetric heat capacity, *C,* on the y-axis, the predetermined series of time points, *tᵢ*, on the z-axis, and the pre-computed values of temperature difference, Δ*T,* stored as data points in the three-dimensional array.

11. The computer-implemented method of any preceding claim, wherein the step of using a solution space to generate a calculated temperature difference, Δ*T̂,* for the estimated values of thermal conductivity, *k,* and volumetric heat capacity, *C,* for each of a predetermined series of time points, *tᵢ*, of the solution space comprises:
using a two-dimensional interpolator to interpolate the pre-computed values of temperature difference, Δ*T,* stored as data points to find the calculated temperature difference, Δ*T̂,* at each of a predetermined series of time points, *tᵢ*.

12. A computer apparatus configured to perform the method of any preceding claim.

13. A system comprising the computer apparatus of claim 12 and a heat flow penetrometer.

14. A computer-readable medium comprising instructions which, when executed by a computer, cause the computer to carry out the method of any of claims 1-11.

15. A computer program comprising instructions which, when the program is executed by a computer, cause the computer to carry out the method of any of claims 1-11.

## Patentansprüche

1. Computerimplementiertes Verfahren (900) zur Bestimmung der unterirdischen Wärmeleitfähigkeit von Zielboden, wobei das Verfahren umfasst:
Empfangen (902), von einem Wärmefluss-Penetrometer, das von Zielboden umgeben ist, von Temperaturdaten, die eine Temperatur T zu jedem Zeitpunkt tⱼ von n Zeitpunkten umfassen;
Bestimmen (904), für jeden Zeitpunkt tⱼ, einer gemessenen Temperaturdifferenz ΔT;
basierend auf geschätzten Werten von Wärmeleitfähigkeit k und volumenbezogener Wärmekapazität C des Zielbodens, Verwenden eines Lösungsraumes (906), um eine berechnete Temperaturdifferenz ΔT̂ für die geschätzten Werte von Wärmeleitfähigkeit k und volumenbezogener Wärmekapazität C für jeden von einer vorbestimmten Reihe von Zeitpunkten tᵢ des Lösungsraumes zu erzeugen,
wobei der Lösungsraum umfasst:
mehrere Werte der Wärmeleitfähigkeit k;
mehrere Werte der volumenbezogenen Wärmekapazität C; und vorberechnete Werte der Temperaturdifferenz ΔT für jede Kombination von Werten von Wärmeleitfähigkeit k und volumenbezogener Wärmekapazität C und für jeden der vorbestimmten Reihe von Zeitpunkten tᵢ des Lösungsraumes;
Vergleichen (908) der gemessenen Temperaturdifferenz ΔT und der berechneten Temperaturdifferenz ΔT̂ für jeden Zeitpunkt tᵢ des Lösungsraumes, um einen Fehlerwert für die geschätzten Werte von Wärmeleitfähigkeit k und volumenbezogener Wärmekapazität C zu bestimmen;
Korrigieren (910) der geschätzten Werte von Wärmeleitfähigkeit k und volumenbezogener Wärmekapazität C des Zielbodens basierend auf dem Fehlerwert; und
Ausgeben (912) der korrigierten geschätzten Werte von Wärmeleitfähigkeit k und volumenbezogener Wärmekapazität C.

2. Computerimplementiertes Verfahren nach Anspruch 1, welches ferner den Schritt des Normalisierens jeder gemessenen Temperaturdifferenz ΔT basierend auf einem Referenzleistungspegel Q_{ref} und einem Leistungspegel, bei dem die Temperaturdaten erzeugt werden, Q_{data}, umfasst.

3. Computerimplementiertes Verfahren nach einem von Anspruch 1 oder Anspruch 2, wobei die geschätzten Werte von Wärmeleitfähigkeit k und volumenbezogener Wärmekapazität C unter Verwendung eines Minimierungs-Solvers korrigiert werden, um den Fehlerwert zu minimieren.

4. Computerimplementiertes Verfahren nach Anspruch 3, wobei eine Regularisierungsfunktion zu dem Minimierungs-Solver hinzugefügt wird, welche eine Strafe vorsieht, wenn eine korrigierte geschätzte volumenbezogene Wärmekapazität C erzeugt wird, die sich von einer vorbestimmten volumenbezogenen Wärmekapazität C unterscheidet.

5. Computerimplementiertes Verfahren nach einem der vorhergehenden Ansprüche, wobei die vorberechneten Werte der Temperaturdifferenz T unter Verwendung eines numerischen Vorwärts-Solvers vorberechnet werden.

6. Computerimplementiertes Verfahren nach einem der vorhergehenden Ansprüche, wobei die vorberechneten Werte der Temperaturdifferenz ΔT basierend auf mindestens einem geometrischen Merkmal des Wärmefluss-Penetrometers vorberechnet werden.

7. Computerimplementiertes Verfahren nach einem der vorhergehenden Ansprüche, wobei die vorberechneten Werte der Temperaturdifferenz ΔT basierend auf einer thermischen Eigenschaft des Wärmefluss-Penetrometers vorberechnet werden.

8. Computerimplementiertes Verfahren nach einem der vorhergehenden Ansprüche, welches ferner den Schritt des Abgleichens der gemessenen Temperaturdifferenz ΔT mit dem Lösungsraum basierend auf der vorbestimmten Reihe von Zeitpunkten tᵢ des Lösungsraumes und unter Verwendung einer Interpolation umfasst.

9. Computerimplementiertes Verfahren nach einem der vorhergehenden Ansprüche, wobei der Fehlerwert durch Berechnen der Wurzel des mittleren quadratischen Fehlers der gemessenen Temperaturdifferenz ΔT und der berechneten Temperaturdifferenz ΔT̂ für jeden Zeitpunkt tᵢ des Lösungsraumes bestimmt wird.

10. Computerimplementiertes Verfahren nach einem der vorhergehenden Ansprüche, wobei der Lösungsraum ein dreidimensionales Array mit den mehreren Werten der Wärmeleitfähigkeit k auf der x-Achse, den mehreren Werten der volumenbezogenen Wärmekapazität C auf der y-Achse, der vorbestimmten Reihe von Zeitpunkten tᵢ auf der z-Achse und den vorberechneten Werten der Temperaturdifferenz ΔT, die als Datenpunkte in dem dreidimensionalen Array gespeichert sind, umfasst.

11. Computerimplementiertes Verfahren nach einem der vorhergehenden Ansprüche, wobei der Schritt des Verwendens eines Lösungsraumes, um eine berechnete Temperaturdifferenz ΔT̂ für die geschätzten Werte von Wärmeleitfähigkeit k und volumenbezogener Wärmekapazität C für jeden von einer vorbestimmten Reihe von Zeitpunkten tᵢ des Lösungsraumes zu erzeugen, umfasst:
Verwenden eines zweidimensionalen Interpolators, um die vorberechneten Werte der Temperaturdifferenz ΔT, die als Datenpunkte gespeichert sind, zu interpolieren, um die berechnete Temperaturdifferenz ΔT̂ an jedem von einer vorbestimmten Reihe von Zeitpunkten tᵢ zu finden.

12. Computervorrichtung, welche dafür ausgelegt ist, das Verfahren nach einem der vorhergehenden Ansprüche durchzuführen.

13. System, welches die Computervorrichtung nach Anspruch 12 und ein Wärmefluss-Penetrometer umfasst.

14. Computerlesbares Medium, welches Anweisungen umfasst, die, wenn sie von einem Computer ausgeführt werden, den Computer veranlassen, das Verfahren nach einem der Ansprüche 1-11 durchzuführen.

15. Computerprogramm, welches Anweisungen umfasst, die, wenn das Programm von einem Computer ausgeführt wird, den Computer veranlassen, das Verfahren nach einem der Ansprüche 1-11 durchzuführen.

## Revendications

1. Procédé (900) mis en œuvre par ordinateur de détermination de conductivité thermique souterraine d'un sol cible, le procédé comprenant :
la réception (902), en provenance d'un pénétromètre à flux de chaleur qui est entouré par un sol cible, de données de température comprenant une température, T, à chaque instant tⱼ parmi n instants ;
la détermination (904), pour chaque instant, tⱼ, d'une différence de température mesurée, ΔT ;
sur la base de valeurs estimées de conductivité thermique, k, et de capacité calorifique volumique, C, du sol cible, l'utilisation d'un espace (906) de solutions pour générer une différence de température calculée, ΔT̂, pour les valeurs estimées de conductivité thermique, k, et de capacité calorifique volumique, C, pour chacun d'une série prédéterminée d'instants, tᵢ, de l'espace de solutions,
l'espace de solutions comprenant :
une pluralité de valeurs de conductivité thermique, k ;
une pluralité de valeurs de capacité calorifique volumique, C ; et
des valeurs pré-calculées de différence de température, ΔT, pour chaque combinaison de valeurs de conductivité thermique, k, et de capacité calorifique volumique, C, et pour chacun de la série prédéterminée d'instants, tᵢ, de l'espace de solutions ;
la comparaison (908) de la différence de température mesurée, ΔT, et de la différence de température calculée, ΔT̂, pour chaque instant, tᵢ, de l'espace de solutions afin de déterminer une valeur d'erreur pour les valeurs estimées de conductivité thermique, k, et de capacité calorifique volumique, C ;
l'ajustement (910) des valeurs estimées de conductivité thermique, k, et de capacité calorifique volumique, C, du sol cible en fonction de la valeur d'erreur ; et
la sortie (912) des valeurs estimées ajustées de conductivité thermique, k, et de capacité calorifique volumique, C.

2. Procédé mis en œuvre par ordinateur selon la revendication 1, comprenant en outre l'étape de normalisation de chaque différence de température mesurée, ΔT, sur la base d'un niveau de puissance de référence, Q_{ref}, et d'un niveau de puissance auquel les données de température sont générées, Q_{data}.

3. Procédé mis en œuvre par ordinateur selon la revendication 1 ou la revendication 2, les valeurs estimées de conductivité thermique, k, et de capacité calorifique volumique, C, étant ajustées en utilisant un solveur de minimisation pour minimiser la valeur d'erreur.

4. Procédé mis en œuvre par ordinateur selon la revendication 3, une fonction de régularisation étant ajoutée au solveur de minimisation qui fournit une pénalité lorsqu'une capacité calorifique volumique estimée ajustée, C, est générée qui diffère d'une capacité calorifique volumique prédéterminée, C.

5. Procédé mis en œuvre par ordinateur selon l'une quelconque des revendications précédentes, les valeurs pré-calculées de différence de température, ΔT, étant pré-calculées en utilisant un solveur numérique direct.

6. Procédé mis en œuvre par ordinateur selon l'une quelconque des revendications précédentes, les valeurs pré-calculées de différence de température, ΔT, étant pré-calculées sur la base d'au moins une caractéristique géométrique du pénétromètre à flux de chaleur.

7. Procédé mis en œuvre par ordinateur selon l'une quelconque des revendications précédentes, les valeurs pré-calculées de différence de température, ΔT, étant pré-calculées sur la base d'une caractéristique thermique du pénétromètre à flux de chaleur.

8. Procédé mis en œuvre par ordinateur selon l'une quelconque des revendications précédentes, comprenant en outre l'étape de mise en correspondance de la différence de température mesurée, ΔT, avec l'espace de solutions sur la base de la série prédéterminée d'instants, tᵢ de l'espace de solutions et d'utilisation d'une interpolation.

9. Procédé mis en œuvre par ordinateur selon l'une quelconque des revendications précédentes, la valeur d'erreur étant déterminée en calculant la racine de l'erreur quadratique moyenne de la différence de température mesurée,ΔT, et de la différence de température calculée, ΔT̂, pour chaque instant, tᵢ, de l'espace de solutions.

10. Procédé mis en œuvre par ordinateur selon l'une quelconque des revendications précédentes, dans lequel l'espace de solutions comprend un tableau tridimensionnel avec la pluralité de valeurs de conductivité thermique, k, sur l'axe x, la pluralité de valeurs de capacité calorifique volumique, C, sur l'axe y, la série prédéterminée d'instants, tᵢ, sur l'axe z, et les valeurs pré-calculées de différence de température, ΔT, stockées en tant que points de données dans le tableau tridimensionnel.

11. Procédé mis en œuvre par ordinateur selon l'une quelconque des revendications précédentes, l'étape d'utilisation d'un espace de solutions pour générer une différence de température calculée, ΔT̂, pour les valeurs estimées de conductivité thermique, k, et de capacité calorifique volumique, C, pour chacun d'une série prédéterminée d'instants, tᵢ, de l'espace de solutions comprenant :
l'utilisation d'un interpolateur bidimensionnel pour interpoler les valeurs pré-calculées de différence de température, ΔT, stockées en tant que points de données pour trouver la différence de température calculée, ΔT̂, à chacun d'une série prédéterminée d'instants, tᵢ.

12. Appareil informatique configuré pour réaliser le procédé selon l'une quelconque des revendications précédentes.

13. Système comprenant l'appareil informatique selon la revendication 12 et un pénétromètre à flux de chaleur.

14. Support lisible par ordinateur comprenant des instructions qui, lorsqu'elles sont exécutées par un ordinateur, amènent l'ordinateur à mettre en œuvre le procédé selon les revendications 1 à 11.

15. Programme informatique comprenant des instructions qui, lorsque le programme est exécuté par un ordinateur, amènent l'ordinateur à mettre en œuvre le procédé selon les revendications 1 à 11.
